# EUROPEAN PATENT APPLICATION

(11) **EP 1 647 601 A1**
(43) Date of publication of application: **19.04.2006**
(21) Application number: 05256312.9
(22) Date of filing: 11.10.2005
(51) Int. Cl.: C12Q 1/68

(54) **Array-based comparative genome hybridization assays**

(30) Priority: 12.10.2004 US 964504
(71) Applicant: Agilent Technologies, Inc., Palo Alto, CA 94306 (US)
(72) Inventor: Curry, Bo U., Redwood City, CA 94061 (US); Anderson, Paige L., Belmont, CA 94002 (US)
(74) Representative: Tollett, Ian

(57) **Abstract**

Methods and compositions for performing array-based comparative genome hybridization (CGH) assays are provided. In general, the methods involve evaluating copy number of a genomic region by evaluating binding of distinguishably labeled populations of nucleic acids to a first CGH array in which: a) the populations of nucleic acids are labeled by a labeled primer and/or b) binding is assessed relative to binding of dye-swapped populations of nucleic acids to a second CGH array. Kits and systems for use in practising the subject methods are also provided.

## Description

This application relates to array-based comparative genome hybridization assays, and, in particular, to methods and compositions for reducing label variation in such assays.

Variations in the copy number of genomic sequences are associated with a variety of diseases and conditions. For example, gains and losses of genomic sequences up to and including whole chromosomes occur in many malignancies, e.g., colon cancer (Rajagopalan et al., Nature Cancer Review (2003) 3:695-701; Rabinovitch et al, Cancer Res. (1999) 59:5148-5153). As a result, tumor cells frequently have aneuploid genomes containing variable numbers of chromosomes and genetic content that deviates significantly from the normal diploid DNA content of non-neoplastic cells. Furthermore, genetic disorders frequently result from loss or gain of chromosomal regions. For example, in humans, trisomy of chromosome 21 results in Down's syndrome, trisomy of chromosome 13 results in Patau syndrome and abnormal numbers of sex chromosomes result in various developmental disorders, while abnormalities on the long arm of chromosome 18 are associated with 18q deletion syndrome.

Comparative genomic hybridization (CGH) is one approach that has been employed to evaluate variations in copy number of genomic regions in cells. In one implementation of CGH, genomic DNA is isolated from normal reference cells, as well as from test cells (e.g., tumor cells). The two nucleic acids are differentially labeled and then simultaneously hybridized *in situ* to metaphase chromosomes of a reference cell. Chromosomal regions in the test cells which are at increased or decreased copy number can be identified by detecting regions where the ratio of signal from the two distinguishably labeled nucleic acids is altered. For example, those regions that have been decreased in copy number in the test cells will show relatively lower signal from the test nucleic acid than the reference compared to other regions of the genome. Regions that have been increased in copy number in the test cells will show relatively higher signal from the test nucleic acid.

In a recent variation of the above traditional CGH approach, the immobilized chromosome element has been replaced with a collection of solid support surface-bound polynucleotides, e.g., an array of surface-bound BAC, cDNA or oligonucleotide probes for regions of a genome. Such approaches offer benefits over immobilized chromosome approaches, including a higher resolution, as defined by the ability of the assay to localize chromosomal alterations to specific areas of the genome.

However, despite the success of array-based CGH assays, the observed results obtained from such assays do not always accurately reflect the actual abundance of genomic regions in the sample. This difference between observed and expected results, termed herein as "signal bias" can, under certain circumstances, cause an array-based CGH assay to yield inaccurate results.

Accordingly, improved methods of reducing signal bias in array-based CGH assays are needed in order to more accurately and reliably evaluate copy number of a chromosomal region in a cell. This invention meets this, and other, needs.

### Relevant Literature

United States Patents of interest include: 6,465,182; 6,335,167; 6,251,601; 6,210,878; 6,197,501; 6,159,685; 5,965,362; 5,830,645; 5,665,549; 5,447,841 and 5.348,855. Also of interest are published United States Application 2002/0006622 and published PCT application WO 99/23256. Articles of interest include: Pollack et al. (Proc. Natl. Acad. Sci. (2002) 99: 12963-12968); Wilhelm et al. (Cancer Res. (2002) 62: 957-960); Pinkel et al. (Nat. Genet. (1998) 20: 207-211); Cai et al. (Nat. Biotech. (2002) 20: 393-396); Snijders et al. (Nat. Genet. (2001) 29:263-264); Hodgson et al. (Nat. Genet. (2001) 29:459-464); Trask (Nat. Rev. Genet. (2002) 3: 769-778); Rabinovitch et al. Cancer Res. (1999) 59:5148-5153); and Lee et al. (Human Genet. (1997) 100:291:304).

According to a first aspect of the present invention, there is provided a method for comparing the copy number of at least one nucleic acid sequence in at least two genomic sources as claimed in claim 1.

According to a second aspect of the present invention, there is provided a kit as claimed in claim 8.

According to a third aspect of the present invention there is provided a system for comparative genome hybridization analysis as claimed in claim 9.

Methods and compositions for performing array-based CGH assays are provided. In general, the methods involve evaluating copy number of a genomic region by evaluating binding of distinguishably labeled populations of nucleic acids to a first CGH array in which: a) the populations of nucleic acids are labeled by a labeled primer and/or b) binding is assessed relative to binding of dye-swapped populations of nucleic acids to a second CGH array. The subject methods and compositions may be used to assess copy number of a genomic region, and, as such, may be employed in a variety of diagnostic and research applications. Kits and systems for use in practicing the subject methods are also provided.

Embodiments of the present invention are described below, by way of example only, and with reference to the accompanying drawings, in which:
Fig. 1 is a schematic representation of an exemplary embodiment of the subject invention;
Fig. 2 is a schematic representation of an second exemplary embodiment of the subject invention;
Fig. 3 is a graph showing distributions of the log2 ratios of signals from male target DNA to female target DNA. The curve on the left is the distribution of log2 ratios of 1337 validated probes complementary to regions of chromosome X. The curve on the right is the distribution of log2 ratios of 1632 probes complementary to other chromosomes;
Fig. 4 is a graph showing the log2 ratios of the average signals from two dye-swapped arrays. Samples were labeled using processive incorporation of labeled dUTP. The curve on the left shows the distribution of X-chromosome probes, and curve on the right shows the distribution of autosomal probes;
Fig. 5 is a graph showing distributions of the log2 ratios of signals from male target DNA to female target DNA. The samples were labeled using end-labeled random 8-mer primers, and extensions with unlabeled monomers; and
Fig. 6 is a graph showing distributions of the log2 ratios of the average signals from two dye-swapped arrays. Samples were labeled by priming with end-labeled random 8-mers. The curve on the left shows the distribution of X-chromosome probes, and the curve on the right is the distribution of autosomal probes.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids that are C- or N-glycosides of a purine or pyrimidine base, polypeptides (proteins), polysaccharides (starches, or polysugars), and other chemical entities that contain repeating units of like chemical structure.

The term "nucleic acid" and "polynucleotide" are used interchangeably herein to describe a polymer of any length composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length. Oligonucleotides are usually synthetic and, in many embodiments, are under 50 nucleotides in length.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "surface-bound polynucleotide" refers to a polynucleotide that is immobilized on a surface of a solid substrate, where the substrate can have a variety of configurations, e.g., a sheet, bead, or other structure. In certain embodiments, the collections of oligonucleotide target elements employed herein are present on a surface of the same planar support, e.g., in the form of an array.

The phrase "labeled population of nucleic acids" refers to mixture of nucleic acids that are detectably labeled, e.g., fluorescently labeled, such that the presence of the nucleic acids can be detected by assessing the presence of the label. A labeled population of nucleic acids is "made from" a chromosome source, the chromosome source is usually employed as template for making the population of nucleic acids.

The term "array" encompasses the term "microarray" and refers to an ordered array presented for binding to nucleic acids and the like.

An "array," includes any one-dimensional, two-dimensional, substantially two-dimensional or three dimensional arrangement of spatially addressable regions bearing nucleic acids, particularly oligonucleotides or synthetic mimetics thereof, and the like. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm² or even less than 10 cm², e.g., less than about 5cm², including less than about 1 cm², less than about 1 mm², e.g., 100 µm², or even smaller. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm², or even less than 50 cm², 5 cm², 1 cm², 0.5 cm², or 0.1 cm². In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case of *in situ* fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

An array is "addressable" when it has multiple regions of different moieties (e.g., different oligonucleotide sequences) such that a region (i.e., a "feature" or "spot" of the array) at a particular predetermined location (i.e., an "address") on the array will detect a particular sequence. Array features are typically, but need not be, separated by intervening spaces. In the case of an array in the context of the present application, the "population of labeled nucleic acids" will be referenced as a moiety in a mobile phase (typically fluid), to be detected by "surface-bound polynucleotides" which are bound to the substrate at the various regions. These phrases are synonymous with the terms "target" and "probe", or "probe" and "target", respectively, as they are used in other publications.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental conditions. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1% SDS at 65°C, both with a wash of0.2xSSC and 0.1% SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M NaHPO₄ 7% sodium dodecyl sulfate (SDS), 1 mM EDTA at 65°C, and washing in 0.1×SSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions may affect the degree to which nucleic acids are specifically hybridized to complementary probes. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2×SSC at a temperature of at least about 50°C or about 55°C to about 60°C for about 1 to about 20 minutes; or, multiple washes with a solution with a salt concentration of about 0.1×SSC containing 0.1% SDS at 20 to 50°C for 1 to 15 minutes; or, equivalent conditions. Stringent conditions for washing can also be, e.g., 0.2×SSC/0.1% SDS at 42°C. In instances wherein the nucleic acid molecules are deoxyoligonucleotides (i.e., oligonucleotides), stringent conditions can include washing in 6xSSC/0.05% sodium pyrophosphate at 37°C (for 14-base oligos), 48°C (for 17-base oligos), 55°C (for 20-base oligos), and 60°C (for 23-base oligos). See Sambrook, Ausubel, or Tijssen (cited below) for detailed descriptions of equivalent hybridization and wash conditions and for reagents and buffers, e.g., SSC buffers and equivalent reagents and conditions.

A specific example of stringent assay conditions is rotating hybridization at 65°C in a salt based hybridization buffer with a total monovalent cation concentration of 1.5M (e.g., as described in U.S. Patent Application No. 09/655,482 filed on September 5, 2000, the disclosure of which is herein incorporated by reference) followed by washes of 0.5×SSC and 0.1×SSC at room temperature and 37°C.

Stringent hybridization conditions may also include a "prehybridization" of aqueous phase nucleic acids with complexity-reducing nucleic acids to suppress repetitive sequences. For example, certain stringent hybridization conditions include, prior to any hybridization to surface-bound polynucleotides, hybridization with Cot-1 DNA, or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not especially distinct. In other words, a mixture is not addressable. To be specific, an array of surface bound polynucleotides, as is commonly known in the art and described below, is not a mixture of capture agents because the species of surface bound polynucleotides are spatially distinct and the array is addressable.

"Isolated" or "purified" generally refers to isolation of a substance (compound, polynucleotide, protein, polypeptide, polypeptide, chromosome, etc.) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well known in the art and include, for example, ion-exchange chromatography, affinity chromatography, flow sorting, and sedimentation according to density.

The term "assessing" and "evaluating" are used interchangeably to refer to any form of measurement, and includes determining if an element is present or not. The terms "determining," "measuring," and "assessing," and "assaying" are used interchangeably and include both quantitative and qualitative determinations. Assessing may be relative or absolute. "Assessing the presence of" includes determining the amount of something present, as well as determining whether it is present or absent.

The term "using" has its conventional meaning, and, as such, means employing, e.g. putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

If a surface-bound polynucleotide "corresponds to" a chromosomal region, the polynucleotide usually contains a sequence of nucleic acids that is unique to that chromosomal region. Accordingly, a surface-bound polynucleotide that corresponds to a particular chromosomal region usually specifically hybridizes to a labeled nucleic acid made from that chromosomal, relative to labeled nucleic acids made from other chromosomal regions.

Methods and compositions for performing array-based CGH assays are provided. In general, the methods involve evaluating copy number of a genomic region by evaluating binding of distinguishably labeled populations of nucleic acids to a first CGH array in which: a) the populations of nucleic acids are labeled by a labeled primer and/or b) binding is assessed relative to binding of dye-swapped populations of nucleic acids to a second CGH array. The subject methods and compositions may be used to assess copy number of a genomic region, and, as such, may be employed in a variety of diagnostic and research applications. Kits and systems for use in practicing the subject methods are also provided.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing the invention components that are described in the publications that might be used in connection with the presently described invention.

In certain array-based CGC assays, it is desirable to detect relatively small differences in copy number of a particular chromosomal region in two genomic samples. For example, in certain array-based CGH assays it is desirable to detect chromosomal regions having a relative copy number ratio of 1:2 (i.e., 0.5) or 2:3 (i.e., 0.67). In these assays, relatively small amounts of signal variation (i.e., signal bias) can have a significant effect on results. In theory, a consistent signal variation of 50% (i.e., an actual signal that is consistently 50% greater or less than its theoretical signal) may lead to the conclusion that a particular region of a chromosome is not present or is duplicated in one of the chromosomes of an otherwise diploid cell. Accordingly, array-based CGH assays are highly sensitive to signal variation.

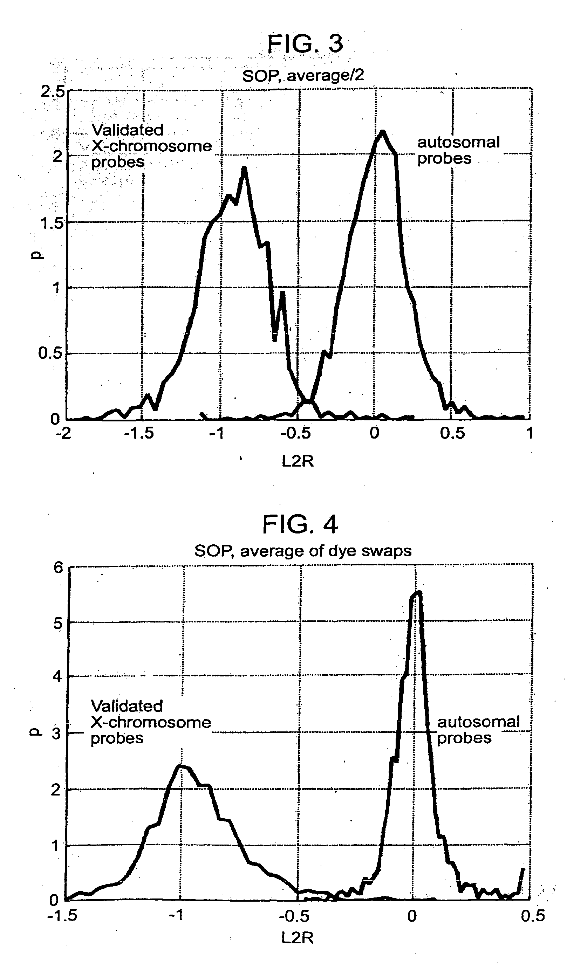

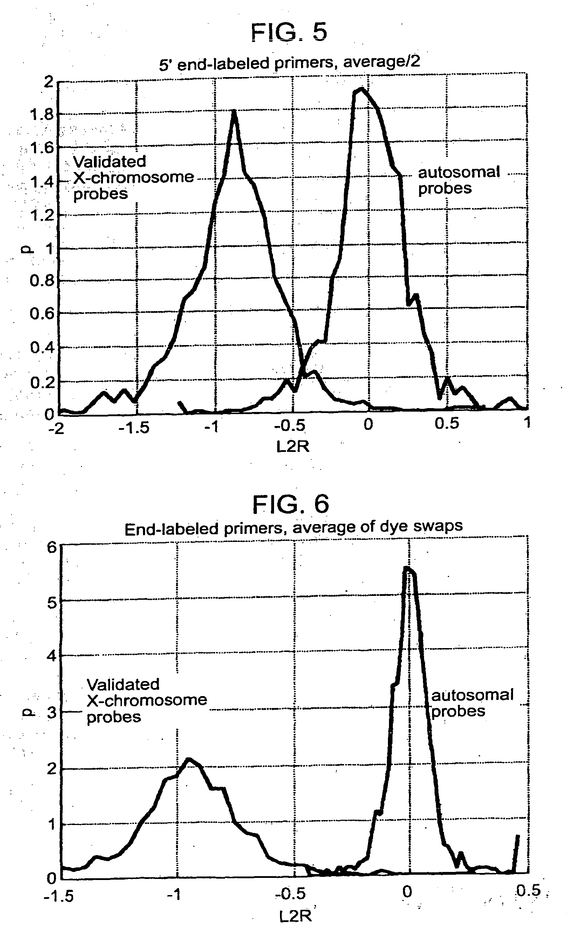

As discussed briefly above, the subject assay methods involve labeling a genomic source using labeled oligonucleotides (e.g., 5' end labeled nucleotides) and/or comparing results of an assay using a first array to those obtained using dye-swapped populations of labeled nucleic acids and a second array. As shown in Figs. 3-6, each of these methods, independently or combined, significantly reduce signal variation, and, as such, provide significantly improved results over prior art methods. Accordingly, the instant disclosure represent a significant contribution to the art.

In further describing the invention in greater detail than that set forth above, the subject 5'-end labeling primer extension methods will be described first, followed by a description of subject dye swapping methods. Finally, representative kits and systems for use in practicing the subject methods will be discussed.

### LABELING METHODS

As discussed above, in particular embodiments of the subject methods genomic sources may be labeled by contacting the genomic source with a labeled oligonucleotide primer under primer extension conditions. These methods produce a 5'end labeled population of nucleic acids for use in an array-based CGH assay.

Genomic sources, primers and primer extension conditions for employment in the subject methods are described in greater detail below.

### Genomic sources

The subject methods involve labeling a genomic source. In many embodiments a "genomic source" is a composition representing the nuclear genome of a cell, or a nucleic acid derivative thereof. For example, a genomic source may contain the entire complement of chromosomes of a cell (i.e., the chromosomes that make up the nuclear genome of a cell), fragmented versions thereof, or amplified fragments thereof. In particular embodiments, therefore, a genomic source contains amplified regions of a cellular genome, e.g., regions that hybridize to particular surface-bound polynucleotides. Many genomic sources are generally well known in the art since they have been described in other methods related to those described herein.

In certain embodiments, a genomic source may be of reduced complexity (usually at least at 20-fold less e.g., 25-fold less, at least about 50-fold less, at least about 75-fold less, at least about 90-fold less, or at least about 95-fold less complex in terms of total numbers of sequences present in the chromosome composition, as compared to the entire chromosome complement of a cell, up to and including a single gene regions (e.g., introns and exons) being represented in the composition), as compared to the entire complement of chromosomes of a cell. In other words, in certain embodiments certain genomic sequences (e.g., repetitive and/or structural sequences) may be removed from a genome by affinity or other means, prior to initiation of the subject labeling methods. In other embodiments, particular genomic regions of interest may be amplified by known methods prior to initiation of the subject methods. In other embodiments, the genomic source does not have reduced (i.e., has non-reduced) complexity.

In general, the cell used in the subject methods may be any cell of interest, including any cell that contains or is suspected of containing a genomic region having abnormal copy number. Accordingly, cells from yeast, plants and animals, such as fish, birds, reptiles, amphibians and mammals may be used in the subject methods. In certain embodiments, mammalian cells, i.e., cells from mice, rabbits, primates, or humans, or cultured derivatives thereof, may be used.

In certain embodiments, a genomic source may represent a complex genome of at least about 1×10⁸ base pairs, including at least about 1×10⁹ base pairs, e.g., about 3×10⁹ base pairs. The average size of the constituent molecules that make up a genomic source may vary greatly. In certain embodiments, the constituent molecules have an average size of at least about 1 Mb, where a representative range of sizes is from about 50 Mb to about 250 Mb or more. In other embodiments, the sizes may not exceed about 1 MB, such that they may be about 1 Mb or smaller, e.g., less than about 500 Kb, etc. Since the subject methods involve a cleavage step that will reduce the average size of the constituent molecules in the genomic source, the precise size of the constituent molecules in the genomic source is not critical to the invention. Accordingly, such molecules may be in the region of 1 kb -50 Mb, and may be double or single stranded, depending on which labeling methods is employed.

In certain embodiments, two genomic sources are labeled using the subject methods, and, as will be described in greater detail below, those labeled genomic sources may be compared using an array-based comparative genome hybridization assay. Accordingly genomic sources from a test and reference cell pair may be employed in the subject methods. The test and reference cells of a test and reference cell pair may be any two cells. However, in many embodiments, one cell of the pair has or is suspected of having a different phenotype or genotype compared to the other cell. In a particular embodiment, test and reference cell pairs include cancerous cells, e.g., cells that exhibit increased genomic instability, and non-cancerous cells, respectively or cells obtained from a sample of tissue from a test subject, e.g., a subject suspected of having a chromosome region copy number abnormality, and cells obtained from a normal, reference subject, respectively. A genomic source may be prepared from a subject, for example a plant or an animal, which subject is suspected of being homozygous or heterozygous for a deletion or amplification of a genomic region.

The genomic source may be prepared using any convenient protocol. In many embodiments, the genomic source is prepared by first obtaining a starting composition of genomic DNA, e.g., a nuclear fraction of a cell lysate, where any convenient means for obtaining such a fraction may be employed and numerous protocols for doing so are well known in the art (see, e.g., the methods for extracting genomic DNA described in Ausubel et al, Short Protocols in Molecular Biology, 3rd ed., Wiley & Sons, 1995 and Sambrook et al, Molecular Cloning: A Laboratory Manual, Third Edition, 2001 Cold Spring Harbor, N.Y.).

The genomic source is, in many embodiments of interest, genomic DNA representing the entire genome from a particular organism, tissue or cell type. However, in certain embodiments the genomic source may comprise a portion of the genome, e.g., one or more specific chromosomes or regions thereof, such as PCR amplified regions produced with a pairs of specific primers.

### Primers

As discussed above, certain embodiments of the subject methods involve contacting a genomic source with a labeled oligonucleotide primer under primer extension conditions.

The primers employed in these methods may be random primers i.e., primers of randomized sequence (e.g., random 3-mers, random 4-mers, random 5-mers, random 6-mers, random 7-mers or random 8-mers, for example) or, in certain embodiments, a mixture of two or more (e.g., 10 or more, 50 or more, 100 or more, 500 or more or 100 or more, usually up to about 1000 or more) different primers having pre-determined nucleotide sequences. If pre-determined primers are used, they usually correspond to (i.e., base pair with and prime nucleic acid synthesis at) regions of interest in a chromosome in a target cell.

In general, the primers employed in the subject methods may vary in length, and in many embodiments range in length from about 3 to about 25 nt, sometimes from about 5 to about 20 nt and sometimes from about 5 to about 10 nt.

The primers employed in certain embodiments of the subject methods are labeled in that they contain a detectable label covalently linked (directly or indirectly) to any one, two, three or four or more nucleotides. In certain embodiments, a 3' or 5' terminal nucleotide of the primer is labeled. In one embodiment, a 5' end-labeled oligonucleotide is employed. Labels that find use in the subject invention include fluorescent labels, i.e., labels that a contain a fluorophore moiety. Specific fluorescent labels of interest include: xanthene dyes, e.g. fluorescein and rhodamine dyes, such as fluorescein isothiocyanate (FITC), 6-carboxyfluorescein (commonly known by the abbreviations FAM and F),6-carboxy-2',4',7',4,7-hexachlorofluorescein (HEX), 6-carboxy-4', 5'-dichloro-2', 7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G⁵ or G⁵), 6-carboxyrhodamine-6G (R6G⁶ or G⁶), and rhodamine 110; cyanine dyes, e.g. Cy3, Cy5 and Cy7 dyes; coumarins, e.g umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes, ALEXA dyes and quinoline dyes. Specific fluorophores of interest that are commonly used in subject applications include: Pyrene, Coumarin, Diethylaminocoumarin, FAM, Fluorescein Chlorotriazinyl, Fluorescein, R110, Eosin, JOE, R6G, Tetramethylrhodamine, TAMRA, Lissamine, ROX, Napthofluorescein, Texas Red, Napthofluorescein, Cy3, and Cy5, etc. Such labels are generally covalently linked to the 5' terminal nucleotide. Nucleotides labeled covalently linked to fluorescent labels are well known in the art. Further fluorescent labels suitable for use in the subject methods may be found in Kricka et al. (Ann Clin Biochem. 39:114-29, 2002). Such labels can be incorporated into a primer during synthesis of the primer, or linked to the a primer (e.g., a terminus of a primer) after it has been synthesized.

### Primer extension

Primer extension conditions suitable for use in the subject methods are well known in the art (see, e.g., Ausubel et al, Short Protocols in Molecular Biology, 5th ed., Wiley & Sons, 2002 and Sambrook et al., Molecular Cloning: A Laboratory Manual, Third Edition, (2001) Cold Spring Harbor, N.Y.) and, as such, need not be described herein in any more detail than that set forth below. In general, subject labeled primers, a polymerase and non-labeled nucleotides (e.g., naturally-occurring deoxynucleotides) are combined with a genomic source of interest in a suitable buffer (i.e., a buffer recommended for use with a particular polymerase), and maintained under particular conditions suitable for primer extension. The subject primer extension conditions provide for extension of the primers to provide a population of 5'end labeled nucleic acids.

In the primer extension reactions employed in the subject methods of these embodiments, the genomic source is typically first subjected to strand disassociation conditions, e.g., subjected to a temperature ranging from about 80°C to about 100°C, usually from about 90°C to about 95°C for a period of time, and the resultant disassociated template molecules are then contacted with the primer molecules under annealing conditions, where the temperature of the template and primer composition is reduced to an annealing temperature of from about 20°C to about 80°C, usually from about 37°C to about 65°C. In certain embodiments, a "snap-cooling" protocol is employed, where the temperature is reduced to the annealing temperature, or to about 4°C or below in a period of from about 1s to about 30s, usually from about 5s to about 10s.

The resultant annealed primer/template hybrids are then maintained in a reaction mixture that includes the above-discussed reagents at a sufficient temperature and for a sufficient period of time to produce the desired labeled nucleic acids. Typically, this incubation temperature ranges from about 20°C to about 75°C, usually from about 37°C to about 65°C. The incubation time typically ranges from about 5 min to about 18 hr, usually from about 1hr to about 12 hr.

The subject primer extension methods may employ a DNA polymerase, such as a template-dependent polymerase (e.g., T4 DNA polymerase, Taq polymerase, the Klenow fragment of DNA polymerase I or the like). As would be recognized by one of skill in the art, a wide variety of DNA polymerases employable in the subject methods are available. Suitable reaction conditions for use with these DNA polymerases are supplied with the polymerases upon their purchase, or may be found in the catalogs of any supplier thereof (e.g., New England Biolabs, Beverly, MA Stratagene, La Jolla, CA, or the like).

In certain embodiments, a pair of genomic sources, typically a "test" and "reference" pair of genomic sources, will be labeled with "distinguishable" labels in that the labels that can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafluor555 and Alexafluor647 (Molecular Probes, Eugene, OR), BODIPY V-1002 and BODIPY V1005 (Molecular Probes, Eugene, OR), POPO-3 and TOTO-3 (Molecular Probes, Eugene, OR), fluorescein and Texas red (Dupont, Bostan MA) and POPRO3 TOPRO3 (Molecular Probes, Eugene, OR). Further suitable distinguishable detectable labels may be found in Kricka et al. (Ann Clin Biochem. 39:1 14-29, 2002).

The population of labeled nucleic acids produced by the subject methods may be purified from unextended primers and other undesirable reaction components by known methods, e.g., size exclusion chromatography or the like.

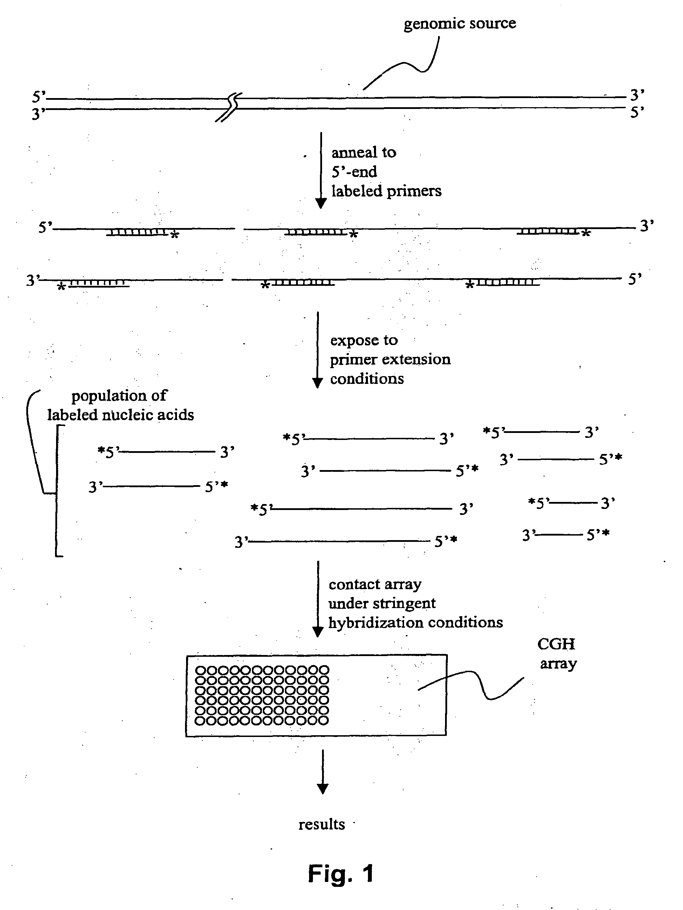

An exemplary CGH assay employing a 5'-end labeled primer labeling methods is illustrated in Fig. 1. According to Fig. 1, a genomic source is annealed to 5'end labeled primers and exposed to primer extension conditions to make a population of labeled nucleic acids. The population of labeled nucleic acids is contacted with a CGH array under stringent hybridization conditions and results are obtained.

In embodiments in which the above-described labeled primer labeling methods are not used, genomic sources may be labeled using any suitable method, e.g., random priming or nick translation, using labeled deoxynucleotides that are incorporated into an nucleic acid product. Such methods are generally well known in the art (see, e.g., Ausubel et al, Short Protocols in Molecular Biology, 3rd ed., Wiley & Sons, 1995 and Sambrook et al, Molecular Cloning: A Laboratory Manual, Third Edition, 2001 Cold Spring Harbor, N.Y.) and, as such, need not be discussed here in any great detail. Exemplary labeling methods that may be used in these embodiments of the invention are described in U.S. Patents 6,465,182; 6,335,167; 6,251,601; 6,210,878; 6,197,501; 6,159,685; 5,965,362; 5,830,645; 5,665,549; 5,447,841 and 5,348,855.

### DYE SWAPPING

As mentioned above, in certain embodiments of the claimed invention, results obtained from an array-based CGH assay may be compared to results obtained using dye swapped populations of the labeled nucleic acids and a second array, where "dye-swapped populations of nucleic acids" are populations of nucleic acids labeled with the same labels as a distinguishably labeled population of nucleic acids, except in reverse. In other words if a first nucleic acid population is labeled with label A and a second nucleic acid population is labeled with label B, then a dye-swapped version of the first and second nucleic acid populations would be labeled with labels B and A, respectively.

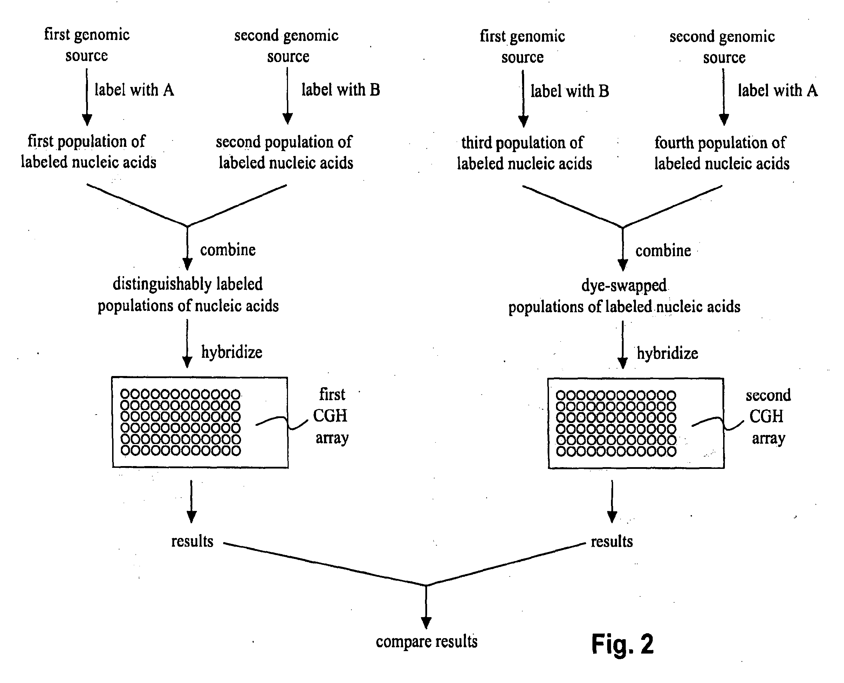

The subject dye swapping methods are most readily described with reference to Fig. 2. With reference to Fig. 2, a first genomic source and a second genomic source are labeled with first and second distinguishable labels (in this case A and B), respectively, to produce first and second populations of distinguishably labeled nucleic acids, respectively. The populations of nucleic acids are combined and hybridized to a first CGH array to provide a first set of results. The first genomic source and the second genomic source are also labeled with the same distinguishable labels (i.e., A and B), except that the first genomic source is labeled with the second label (i.e., B) and the second genomic source is labeled with the first label (i.e., A). In relation to the first and second populations of labeled nucleic acids, the third and fourth populations of nucleic acids are dye swapped populations of nucleic acids. The third and fourth populations of nucleic acids are combined and hybridized to a second CGH array to provide a second set of results. The first and second sets of results are then compared (e.g., averaged) to provide data.

In general, the first array and the second array contain a plurality (e.g., at least 100, at least 1,000, at least 5,000 or at least 10,000) of the same surface-bound polynucleotide features, and, in certain embodiments may contain identical sets of surface-bound polynucleotide features. Accordingly, results obtained from the first array may be directly comparable (after any normalization procedures that may be performed) to results obtained from the second array.

The results obtained from the first and second arrays may be compared using any suitable method. In many embodiments, the results from the first array may be expressed as a series of ratios, one ratio per feature of the array. As is well known in the art, such a ratio represents the relative level of signal from two labels associated with a single feature. The relative level of signal represents the relative level of two chromosomal regions in a genomic source. A ratio obtained from a feature of the first array may be compared to the ratio obtained from a corresponding feature of the second array (where corresponding features typically contain identical surface bound amino acid probes), by a variety of methods to provide data, usually a third ratio (including an integer representing a ratio). In one embodiment, results are compared and data is provided by averaging the ratios obtained from corresponding features of the first and second arrays. Since ratios are multiplicative, a geometric average of the ratios (i.e. the square root of the product of the ratios), or, alternatively, the arithmetic average of the logarithms of the ratios (i.e. the sum of the log ratios divided by two) may also be calculated.

By comparing results obtained from the first and second arrays, a third set of results, representing a comparison between the results of the first and second arrays, may be provided. As shown in Figs. 3-6, this third set of results more accurately describes the actual relative abundance of chromosomal regions in two genomic sources, than either of the first and second sets of results (obtained from the first and second arrays respectively).

In certain embodiments, an individual result (e.g., an individual ratio) in the third set of results may include an assessment of the closeness of the ratios compared to provide that result (e.g., the spread of the first and second ratios averaged to provide the third ratio). As would be recognized by one of skill in the art, such as assessment could be expressed as the difference between the first log ratio and the second log ratio, divided by the third log ratio, for example. Third log ratios that are associated with a large spread (e.g., where the difference between the first and second log ratios is greater than about 20%, 30%, 40% or 50% of the third log ratio, for example) may be marked as being not distinguishable from zero or unreliable or of low quality.

### ARRAY-BASED CGH ASSAY METHODS

As mentioned above, the subject methods find particular use in array-based CGH assays. A description of exemplary CGH assays in which the subject methods may be employed is set forth below. For example, a genomic source may be labeled using a labeled primer and/or the results obtained may be compared to those obtained using dye-swapped populations of nucleic acids, as discussed in further detail above.

In generally, use of the subject methods in a CGH assay produces results that more accurately predict the actual copy number of particular nucleic acids in a genomic source (i.e., those nucleic acids that correspond to surface bound polynucleotides present on the array used). A good indication of the noise associated with the cgh copy number measurement is the width of the distribution of the log ratios measured for probes associated with regions of the genome which are known to have identical copy numbers (e.g. regions of chromosome X in normal male or female samples). In a perfect measurement, the measured log ratios of all probes with the same copy number will be equal. In practice, the measurements fall into a statistical distribution centered at the true ratio, and the width of this distribution is a major factor in the discrimination power of the assay. In general, incorporation of the subject methods into an array-based CGH assay reduces the width (i.e., the standard deviation) of log ratio distributions of autosomal probes by greater than 50%, e.g., greater than 55%, greater than 60%, greater than about 65%, greater than about 70%, greater than about 75% or more, typically up to about 80% or 90% or more, as compared to those of prior art array-based CGH assays. Means for calculating variance (i.e., averaging squared deviation of each data point from the mean) are well known in the statistical arts.

### Array platforms

Array platforms for performing the subject methods are generally well known in the art (e.g., see Pinkel et al., Nat. Genet. (1998) 20:207-211; Hodgson et al., Nat. Genet. (2001) 29:459-464; Wilhelm et al., Cancer Res. (2002) 62: 957-960) and, as such, need not be described herein in any great detail.

In general, arrays suitable for use in performing the subject methods contain a plurality (i.e., at least about 100, at least about 500, at least about 1000, at least about 2000, at least about 5000, at least about 10,000, at least about 20,000, usually up to about 100,000 or more) of addressable features containing polynucleotides that are linked to a usually planar solid support via one terminus of the polynucleotide molecules (i.e. either the 3' or 5' end). Features on a subject array usually contain a polynucleotide that hybridizes with, i.e., specifically binds to, genomic sequences from a cell. Accordingly, such ''comparative genome hybridization arrays", for short "CGH arrays" typically have a plurality of different BACs, cDNAs, oligonucleotides, or inserts from phage or plasmids, etc., that are addressably arrayed. In many embodiments, the subject CGH arrays contain single-stranded polynucleotides bound to the surface of a substrate one end of the polynucleotides. As such, CGH arrays usually contain surface bound polynucleotides that are about 10-25 bases in length, 15-80 bases in length, about 20-100 bases in length, about 100-1000 bases in length, or about 200-5000 bases in length, depending on the platform used. Suitable array platforms for CGH experiments, and exemplary methods by which such assays may be performed, are described in issued U.S. patents 6,465,182; 6,335,167; 6,251,601; 6,210,878; 6,197,501; 6,159,685; 5,965,362; 5,830,645; 5,665,549; 5,447,841 and 5,348,855, which patents are incorporated by reference in their entirety.

In particular embodiments, CGH arrays containing surface-bound oligonucleotides, i.e., oligonucleotides of about 10 to about 100 nucleotides and up to about 200 nucleotides in length, find particular use in the subject methods.

### Methods

The methods described above are generally useful in methods of assessing copy number of a genomic region, where a genomic region is generally of any length equal to or greater than the length of a surface bound polynucleotide corresponding to that region. In certain embodiments, genomic regions are greater than about 20 bp, between about 500 bp and the length of an intact chromosome, e.g., any about 1 kb to about 1 Mbp or about 10 kb to about 1000 kb region of a genome. In general, the methods involve contacting a first population of end-labeled nucleic acids made from a genomic source for a test cell with an array of surface-immobilized polynucleotides under conditions that provide for specific hybridization, and evaluating binding of the end-labeled nucleic acids for binding to the surface-immobilized polynucleotides. In certain embodiments, evaluating is done relative to binding of a reference population of end-labeled nucleic acids made from a genomic source for a reference cell.

In general, the subject array CGH assays involve labeling a test and reference genomic source to make two labeled populations of nucleic acids which may be distinguishably labeled, contacting the labeled populations of nucleic acids with at least one array of surface bound polynucleotides under specific hybridization conditions, and analyzing any data obtained from hybridization of the nucleic acids to the surface bound polynucleotides. Such methods are generally well known in the art (see, e.g., Pinkel et al., Nat. Genet. (1998) 20:207-211; Hodgson et al., Nat. Genet. (2001) 29:459-464; Wilhelm et al., Cancer Res. (2002) 62: 957-960)) and, as such, need not be described herein in any great detail. In certain embodiments, the labeled populations of nucleic acids may be contacted to the same array and binding assayed in series. In other words, the first labeled population of nucleic acids may be contacted with an array and results obtained, the array then washed to remove nucleic acids bound to the array, and the second labeled population of nucleic acids then contacted with the array and results obtained.

The above-described labeling reactions produce a first and second population of end-labeled nucleic acids that correspond to the test and reference cells, respectively. After nucleic acid purification and any pre-hybridization steps to suppress repetitive sequences (e.g., hybridization with Cot-1 DNA), the populations of end-labeled nucleic acids are contacted to an array of surface bound polynucleotides, as discussed above, under conditions such that nucleic acid hybridization to the surface bound polynucleotides can occur, e.g., in a buffer containing 50% formamide, 5×SSC and 1% SDS at 42°C, or in a buffer containing 5×SSC and 1% SDS at 65°C, both with a wash of 0.2×SSC and 0.1% SDS at 65°C.

The labeled nucleic acids can be contacted to the surface bound polynucleotides serially, or, in other embodiments, simultaneously (i.e., the labeled nucleic acids are mixed prior to their contacting with the surface-bound polynucleotides). Depending on how the nucleic acid populations are labeled (e.g., if they are distinguishably or indistinguishably labeled), the populations may be contacted with the same array or different arrays. Where the populations are contacted with different arrays, the different arrays are substantially, if not completely, identical to each other in terms of target feature content and organization.

Standard hybridization techniques (using high stringency hybridization conditions) are used to hybridize a sample to a target nucleic acid array. Suitable methods are described in references describing CGH techniques (Kallioniemi et al., Science 258:818-821 (1992) and WO 93/18186). Several guides to general techniques are available, e.g., Tijssen, Hybridization with Nucleic Acid Probes, Parts I and II (Elsevier, Amsterdam 1993). For a descriptions of techniques suitable for in situ hybridizations see, Gall et al. (Meth. Enzymol., 21:470-480 (1981)) and Angerer et al. (in Genetic Engineering: Principles and Methods Setlow and Hollaender, Eds. Vol 7, pgs 43-65 (plenum Press, New York 1985)). See also United States Patent Nos: 6,335, 167; 6,197,501; 5,830,645; and 5,665,549; the disclosures of which are herein incorporate by reference.

Generally, comparative genome hybridization methods comprise the following major steps: (1) immobilization of polynucleotides on a solid support by one end of the polynucleotide; (2) pre-hybridization treatment to increase accessibility of support-bound polynucleotides and to reduce nonspecific binding; (3) hybridization of a mixture of distal end-labeled nucleic acids to the surface-bound nucleic acids, typically under high stringency conditions; (4) post-hybridization washes to remove nucleic acid fragments not tightly bound to the solid support polynucleotides; and (5) detection of the hybridized labeled nucleic acids. The reagents used in each of these steps and their conditions for use vary depending on the particular application.

As indicated above, hybridization is carried out under suitable hybridization conditions, which may vary in stringency as desired. In certain embodiments, highly stringent hybridization conditions may be employed. The term "high stringency hybridization conditions" as used herein refers to conditions that limit nucleic acid binding complexes on an array surface to specifically complementary binding members, i.e., between the surface-bound polynucleotides and complementary labeled nucleic acids in a sample. Representative high stringency assay conditions that may be employed in these embodiments are provided above.

The above hybridization step may include mixing and/or agitation of the immobilized polynucleotides and the sample of labeled nucleic acids, where the agitation may be accomplished using any convenient protocol, e.g., shaking, rotating, spinning, and the like.

Following hybridization, the array-surface bound polynucleotides are typically washed to remove unbound and not tightly bound labeled nucleic acids. Washing may be performed using any convenient washing protocol, where the washing conditions are typically stringent, as described above.

Following hybridization and washing, as described above, the hybridization of the labeled nucleic acids to the targets is then detected using standard techniques so that the surface of immobilized targets, e.g., the array, is read. Reading of the resultant hybridized array may be accomplished by illuminating the array and reading the location and intensity of resulting fluorescence at each feature of the array to detect any binding complexes on the surface of the array. For example, a scanner may be used for this purpose, which is similar to the AGILENT MICROARRAY SCANNER available from Agilent Technologies, Palo Alto, CA. Other suitable devices and methods are described in U.S. patent applications: Serial No. 09/846125 "Reading Multi-Featured Arrays" by Dorsel et al. (published as US2002/0160369 and US 6,756,202); and United States Patent No. 6,406,849, which references are incorporated herein by reference. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). In the case of indirect labeling, subsequent treatment of the array with the appropriate reagents may be employed to enable reading of the array. Some methods of detection, such as surface plasmon resonance, do not require any labeling of nucleic acids, and are suitable for some embodiments.

Results from the reading or evaluating may be raw results (such as fluorescence intensity readings for each feature in one or more color channels) or may be processed results (such as those obtained by subtracting a background measurement, or by rejecting a reading for a feature which is below a predetermined threshold, normalizing the results, and/or forming conclusions based on the pattern read from the array (such as whether or not a particular target sequence may have been present in the sample, or whether or not a pattern indicates a particular condition of an organism from which the sample came).

In certain embodiments, the subject methods include a step of transmitting data or results from at least one of the detecting and deriving steps, also referred to herein as evaluating, as described above, to a remote location. By "remote location" is meant a location other than the location at which the array is present and hybridization occurred. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart.

"Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc. Accordingly, a pair of genomic sources may be labeled to make two populations of end-labeled nucleic acids, the nucleic acids contacted with an array of surface-bound polynucleotides, and the level of labeled nucleic acids bound to each surface-bound polynucleotide is assessed.

In certain embodiments, a surface-bound polynucleotide is assessed by determining the level of binding of the population of labeled nucleic acids to that polynucleotide. The term "level of binding" means any assessment of binding (e.g. a quantitative or qualitative, relative or absolute assessment) usually done, as is known in the art, by detecting signal (i.e., pixel brightness) from the label associated with the labeled nucleic acids. Since the level of binding of labeled nucleic acid to a surface-bound polynucleotide is proportional to the surface density of bound label, the level of binding of labeled nucleic acid is usually determined by assessing the amount of label associated with the surface-bound polynucleotide.

In certain embodiments, a surface-bound polynucleotide may be assessed by evaluating its binding to two populations of nucleic acids that are distinguishably labeled. In these embodiments, for a single surface-bound polynucleotide of interest, the results obtained from hybridization with a first population of labeled nucleic acids may be compared to results obtained from hybridization with the second population of nucleic acids, usually after normalization of the data. The results may be expressed using any convenient means, e.g., as a number or numerical ratio, etc.

By "normalization" is meant that data corresponding to the two populations of nucleic acids are globally normalized to each other, and/or normalized to data obtained from controls (e.g., internal controls produce data that are predicted to equal in value in all of the data groups). Normalization generally involves multiplying each numerical value for one data group by a value that allows the direct comparison of those amounts to amounts in a second data group. Several normalization strategies have been described (Quackenbush et al, (Nat Genet. 32 Suppl:496-501, 2002), Bilban et at (Curr Issues Mol Biol. 4:57-64, 2002), Finkelstein et al, (Plant Mol Biol.48(1-2):119-31, 2002), and Hegde et al, (Biotechniques. 29:548-554, 2000)). Specific examples of normalization suitable for use in the subject methods include linear normalization methods, non-linear normalization methods, e.g., using lowess local regression to paired data as a function of signal intensity, signal-dependent non-linear normalization, qspline normalization and spatial normalization, as described in Workman et al., (Genome Biol. 2002 3, 1-16). In certain embodiments, the numerical value associated with a feature signal is converted into a log number, either before or after normalization occurs. Data may be normalized to data obtained using the data obtained from a support-bound polynucleotide for a chromosome of known concentration in any of the chromosome compositions.

Accordingly, binding of a surface-bound polynucleotide to an end-labeled population of nucleic acids may be assessed. In most embodiments, the assessment provides a numerical assessment of binding, and that numeral may correspond to an absolute level of binding, a relative level of binding, or a qualitative (e.g., presence or absence) or a quantitative level of binding. Accordingly, a binding assessment may be expressed as a ratio, whole number, or any fraction thereof.

In other words, any binding may be expressed as the level of binding of a surface-bound polynucleotide to an end-labeled population of nucleic acids made from a test genomic source, divided by its level of binding to an labeled population of nucleic acids made from a reference genomic source (or vice versa).

Accordingly, the invention also provides a system for performing an array-based CGH assay. In many embodiments, the system contains reagents for labeling a pair of genomic sources to provide distinguishably labeled populations of nucleic acids and dye-swapped labeled populations of nucleic acids, and a CGH array. The components of the subject system depend on the exact method used, and, as such, the components may vary.

### KITS

Also provided by the subject invention are kits for practicing the subject methods, as described above. The subject kits at least include reagents for labeling a pair of genomic sources to provide a distinguishably labeled populations of nucleic acids and dye-swapped labeled populations of nucleic acids. For example, a kit may contain a first and second primers that are identical in sequence but distinguishably labeled. Other optional components of the kit include: at least two CGH arrays. A kit may contain a computer readable medium of the invention, as described in greater detail below. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

In addition to the subject database, programming and instructions, the kits may also include one or more control analyte mixtures, e.g., two or more control analytes for use in testing the kit.

### COMPUTER-RELATED EMBODIMENTS

The invention also provides a variety of computer-related embodiments. Specifically, the invention provides software for performing the comparison between the results obtained from a first array to those obtained from a second array, according to the methods described above. In particular embodiments, the comparison of results described above may be assessed by software (typically executed by a computer processor) to provide a third set of results. The software may be stand-alone, or may be incorporated or incorporatable into other array analysis software.

In many embodiments, the methods are coded onto a computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer.

With respect to computer readable media, "permanent memory" refers to memory that is permanent. Permanent memory is not erased by termination of the electrical supply to a computer or processor. Computer hard-drive ROM (i.e. ROM not used as virtual memory), CD-ROM, floppy disk and DVD are all examples of permanent memory. Random Access Memory (RAM) is an example of non-permanent memory. A file in permanent memory may be editable and re-writable.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

### UTILITY

The subject methods find most application in CGH assays, e.g., any application in which one wishes to compare the copy number of nucleic acid sequences found in two or more genomic samples.

One type of representative application in which the subject CGH arrays find use is the quantitative comparison of copy number of one nucleic acid sequence in a first collection of nucleic acid molecules relative to the copy number of the same sequence in a second collection.

As such, the present invention may be used in methods of comparing abnormal nucleic acid copy number and mapping of chromosomal abnormalities associated with disease. In many embodiments, the subject methods are employed in applications that use polynucleotides immobilized on a solid support, to which differentially labeled nucleic acids produced as described above are hybridized. Analysis of processed results of the described hybridization experiments provides information about the relative copy number of nucleic acid domains, e.g. genes or regions thereof, in genomes.

Such applications compare the copy numbers of sequences capable of binding to the target elements. Variations in copy number detectable by the methods of the invention may arise in different ways. For example, copy number may be altered as a result of amplification or deletion of a chromosomal region, e.g. as commonly occurs in cancer.

Representative applications in which the subject methods find use are further described in U.S. Patent Nos. 6,335,167; 6,197,501; 5,830,645; and 5,665,549; the disclosures of which are herein incorporated by reference.

The following examples are offered by way of illustration and not by way of limitation.

### EXPERIMENTAL

Samples containing 6 µg of denatured genomic DNA were labeled using methods set forth above and hybridized to oligonucleotide arrays. In experiments where labeled primers were used, 20 µg of random 8-mers, labeled with a fluorescent dye (Cy3 or Cy5) at the 5'end, were mixed with denatured genomic DNA in the presence of 1.2 mm of each of G, A, T and C unlabeled nucleotides and polymerase. Results of these experiments are set forth in Figs. 3-6 and described in further detail in the brief description of the figures.

The above results and discussion demonstrate a new method for performing array-based CGH assays. The subject methods are superior to prior art methods because they provide data that is significantly less variable. Since low variability is vital to array-based CGH analysis, the subject methods represent a significant contribution to the art.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application No. 10/964,504, from which this application claims priority, and in the abstract accompanying this application are incorporated herein by reference.

## Claims

1. A method for comparing the copy number of at least one nucleic acid sequence in at least two genomic sources, said method comprising:
(a) preparing a first collection of labeled nucleic acid molecules from a first genomic source and a second collection of labeled nucleic acid molecules from said second genomic source;
(b) contacting said first and second collections of labeled nucleic acid molecules with a first nucleic acid array; and
(c) evaluating the binding of the first and second collections of labeled nucleic acid molecules to said array to compare the copy number of at least one nucleic acid sequence in said at least two genomic sources;
wherein said method is further **characterized by** at least one of:
(i) said preparing step (a) employs a labeled primer composition to produce first and second collections of end labeled nucleic acid molecules; and
(ii) said method further comprises comparing results of said evaluating step (c) to results obtained using dye-swapped labeled populations of nucleic acids and a second nucleic acid array.

2. A method as claimed in claim 1, wherein said method is further **characterized by** both of:
(i) said preparing step (a) employs a labeled primer composition to produce first and second collections of end labeled nucleic acid molecules; and
(ii) said method further comprises comparing results of said evaluating step (c) to results obtained using dye-swapped labeled populations of nucleic acids and a second nucleic acid array.

3. A method as claimed in claim 1 or 2, wherein said nucleic acid array is an oligonucleotide array.

4. A method as claimed in any preceding claim, wherein said labeled primer is labeled with a fluorophore.

5. A method as claimed in any preceding claim, wherein said primer composition is a random primer composition.

6. A method as claimed in any preceding claim, wherein said labeled primer contains single label

7. A method as claimed in any preceding claim, wherein said labeled primer is labeled at its 5'end.

8. A kit comprising:
a first labeled oligonucleotide primer and a second labeled oligonucleotide primer, wherein said first and second labeled oligonucleotide primers are identical in sequence and distinguishably labeled, and
instructions for performing the method as claimed in any preceding claim.

9. A system for comparative genome hybridization (CGH) analysis, comprising:
a) a first labeled primer and a second labeled primer, wherein said primers are identical in sequence and distinguishably labeled; and
b) at least two identical CGH arrays.

10. A system as claimed in claim 9, wherein said primers are random primers.
